# EUROPEAN PATENT APPLICATION

(11) **EP 3 878 946 A1**
(43) Date of publication of application: **15.09.2021**
(21) Application number: 19881986.4
(22) Date of filing: 15.10.2019
(51) Int. Cl.: C12N 5/071, C12N 5/077, C12N 5/0775, C12N 15/12, C12Q 1/6809

(54) **HAIR FOLLICLE GERMS, METHOD FOR PRODUCING HAIR FOLLICLE GERMS, AND METHOD FOR ACTIVATING CELLS INCLUDED IN HAIR FOLLICLE GERMS**

(30) Priority: 08.11.2018 JP 2018210524
(71) Applicant: NATIONAL UNIVERSITY CORPORATION YOKOHAMA NATIONAL UNIVERSITY, Yokohama-shi Kanagawa 240-8501 (JP); KANAGAWA INSTITUTE OF INDUSTRIAL SCIENCE AND TECHNOLOGY, Ebina-shi Kanagawa 243-0435 (JP)
(72) Inventor: FUKUDA, Junji, Yokohama-shi, Kanagawa 240-8501 (JP); KAGEYAMA, Tatsuto, Ebina-shi, Kanagawa 243-0435 (JP); TATE, Yoshiki, Yokohama-shi, Kanagawa 240-8501 (JP)
(74) Representative: Winter, Brandl - Partnerschaft mbB
(86) International application number: PCT/JP2019/040444
(87) International publication number: WO 2020/095631

(57) **Abstract**

Provided are a hair follicle germ having excellent hair growth-related properties, a method of producing a hair follicle germ, and a method of activating cells contained in a hair follicle germ. The hair follicle germ includes: epithelial cells; mesenchymal cells; and mesenchymal stem cells. The method of producing a hair follicle germ includes co-culturing epithelial cells, mesenchymal cells, and mesenchymal stem cells to form a hair follicle germ.

## Description

### Technical Field

The present invention relates to a hair follicle germ, a method of producing a hair follicle germ, and a method of activating cells contained in a hair follicle germ.

### Background Art

Progress is being made in development of a technology involving forming hair follicle germs in vitro and transplanting the hair follicle germs to a living body to regenerate hair.

In Patent Literature 1, there is a description of a regenerated hair follicle germ aggregation manufacturing method, including a step of forming hair follicle germs by inoculating a microwell plate, which includes regularly arranged microwell portions, with mesenchymal cells and epithelial cells and culturing a mixture of the cells while supplying oxygen thereto.

In Patent Literature 2, there is a description of a method of producing a cell mass capable of serving as a primitive organ-like structure formed of a plurality of somatic cell types of somatic origin, including: preparing cultures containing the plurality of types of somatic cells; mixing the plurality of types of somatic cell cultures followed by adding a Wnt signal activator to the mixed cell culture; subjecting the culture containing the Wnt signal activator to non-plate contact culturing over a predetermined time period; and replacing the medium of the culture cultured by the non-plate contact culturing with a medium not containing the Wnt signal activator and further culturing for a predetermined time period, wherein, at least one type of the plurality of somatic cells is maintained in an undifferentiated state.

### Citation List

### Patent Literature

[PTL 1] WO 2017/073625 A1
[PTL 2] JP 2013-078344 A

### Summary of Invention

### Technical Problem

However, a method of producing a hair follicle germ having sufficient hair growth-related properties has not yet been established.

The present invention has been made in view of the above-mentioned problem, and one of the objects of the present invention is to provide a hair follicle germ having excellent hair growth-related properties, a method of producing a hair follicle germ, and a method of activating cells contained in a hair follicle germ.

### Solution to Problem

In order to achieve the above-mentioned object, according to one embodiment of the present invention, there is provided a hair follicle germ, including: epithelial cells; mesenchymal cells; and mesenchymal stem cells. According to the one embodiment of the present invention, a hair follicle germ having excellent hair growth-related properties is provided.

In the hair follicle germ, the mesenchymal stem cells may be adipose-derived mesenchymal stem cells. In the hair follicle germ, the mesenchymal cells may be dermal papilla cells and/or dermal sheath cup cells. The hair follicle germ may be a hair follicle germ spheroid.

In the hair follicle germ, a ratio of the number of the mesenchymal stem cells to a total of the number of the epithelial cells and the number of the mesenchymal cells may be 0.01 or more. In the hair follicle germ, a ratio of the number of the mesenchymal stem cells to the number of the epithelial cells may be 0.02 or more. In the hair follicle germ, a ratio of the number of the mesenchymal stem cells to the number of the mesenchymal cells may be 0.02 or more.

In order to achieve the above-mentioned object, according to one embodiment of the present invention, there is provided a method of producing a hair follicle germ, including co-culturing epithelial cells, mesenchymal cells, and mesenchymal stem cells to form a hair follicle germ. According to the one embodiment of the present invention, a method of producing a hair follicle germ having excellent hair growth-related properties is provided.

In the method, the co-culturing may include co-culturing the epithelial cells, the mesenchymal cells, and the mesenchymal stem cells on a non-cell-adhesive surface.

In the method, the hair follicle germ formed by the co-culturing may contain the mesenchymal cells that have been activated compared to mesenchymal cells contained in a hair follicle germ formed by the same method except for omitting use of the mesenchymal stem cells. In this case, in the activated mesenchymal cells, an expression amount of at least one hair growth-related gene may be increased compared to that in mesenchymal cells contained in a hair follicle germ formed by the same method except for omitting use of the mesenchymal stem cells.

In order to achieve the above-mentioned object, according to one embodiment of the present invention, there is provided a method of activating mesenchymal cells, including performing, in a hair follicle germ formed by co-culturing epithelial cells and mesenchymal cells, the co-culture with further addition of mesenchymal stem cells, to thereby activate the mesenchymal cells contained in the hair follicle germ. According to the one embodiment of the present invention, a method of activating mesenchymal cells, for effectively improving the hair growth-related properties of mesenchymal cells, is provided.

### Advantageous Effects of Invention

According to the present invention, a hair follicle germ having excellent hair growth-related properties, a method of producing a hair follicle germ, and a method of activating cells contained in a hair follicle germ, are provided.

### Brief Description of Drawings

FIG. 1A is an explanatory diagram showing a fluorescence micrograph on day 1 of culture of a hair follicle germ produced in Example 1 according to one embodiment of the present invention.
FIG. 1B is an explanatory diagram showing a fluorescence micrograph on day 2 of culture of the hair follicle germ produced in Example 1 according to one embodiment of the present invention.
FIG. 1C is an explanatory diagram showing a fluorescence micrograph on day 3 of culture of the hair follicle germ produced in Example 1 according to one embodiment of the present invention.
FIG. 2A is an explanatory diagram showing a fluorescence micrograph of epithelial cells fluorescently stained in the hair follicle germ on day 3 of culture produced in Example 1 according to one embodiment of the present invention.
FIG. 2B is an explanatory diagram showing a fluorescence micrograph of adipose-derived stem cells fluorescently stained in the hair follicle germ on day 3 of culture produced in Example 1 according to one embodiment of the present invention.
FIG. 2C is an explanatory diagram showing a fluorescence micrograph of epithelial cells, dermal papilla cells, and adipose-derived stem cells fluorescently stained in the hair follicle germ on day 3 of culture produced in Example 1 according to one embodiment of the present invention.
FIG. 3A is an explanatory diagram showing the results of evaluation of Versican gene expression amounts in hair follicle germs in Example 2 according to one embodiment of the present invention.
FIG. 3B is an explanatory diagram showing the results of evaluation of BMP4 gene expression amounts in the hair follicle germs in Example 2 according to one embodiment of the present invention.
FIG. 3C is an explanatory diagram showing the results of evaluation of ALP gene expression amounts in the hair follicle germs in Example 2 according to one embodiment of the present invention.
FIG. 4A is an explanatory diagram showing an example of a phase-contrast micrograph on day 1 of culture of a hair follicle germ produced in Example 3 according to one embodiment of the present invention.
FIG. 4B is an explanatory diagram showing an example of a phase-contrast micrograph on day 3 of culture of the hair follicle germ produced in Example 3 according to one embodiment of the present invention.
FIG. 4C is an explanatory diagram showing another example of a phase-contrast micrograph on day 3 of culture of hair follicle germs produced in Example 3 according to one embodiment of the present invention.
FIG. 5A is an explanatory diagram showing a photograph taken of the state of hair follicle germs formed at a number ratio of epithelial cells:dermal papilla cells:adipose-derived stem cells=1:1:0 and transplanted to a mouse in Example 4 according to one embodiment of the present invention.
FIG. 5B is an explanatory diagram showing a photograph taken of the state of hair follicle germs formed at a number ratio of epithelial cells:dermal papilla cells:adipose-derived stem cells=2:2:1 and transplanted to a mouse in Example 4 according to one embodiment of the present invention.
FIG. 5C is an explanatory diagram showing a photograph taken of the state of hair follicle germs formed at a number ratio of epithelial cells:dermal papilla cells:adipose-derived stem cells=4:4:1 and transplanted to a mouse in Example 4 according to one embodiment of the present invention.

### Description of Embodiments

Now, embodiments of the present invention will be described. The present invention is not limited to these embodiments.

A hair follicle germ according to one embodiment of the present invention includes: epithelial cells; mesenchymal cells; and mesenchymal stem cells. The hair follicle germ is a cell aggregate formed in vitro. For this reason, the hair follicle germ is sometimes called a regenerated hair follicle germ. The hair follicle germ has hair growth-related properties. That is, the hair follicle germ (more specifically, cells contained in the hair follicle germ) expresses a hair growth-related gene. The hair growth-related gene is not particularly limited as long as the gene is related to hair growth (e.g., a gene related to the promotion of hair growth), but may be, for example, one or more selected from the group consisting of Versican, alkaline phosphatase (ALP), BMP4, Nexin, Notch1, Wnt10b, Lymphoid Enhancer Factor-1 (LEF1), Sonic hedgehog (Shh), Msh homeobox 2 (MSX2), and β-Catenin.

In addition, for example, the hair follicle germ causes hair growth when transplanted to a living body. Accordingly, the hair follicle germ is preferably used for transplantation to a living body. That is, the hair follicle germ may be a hair follicle germ for transplantation. The hair follicle germ may be used as it is in any other application, such as research, instead of being used for transplantation.

The epithelial cells contained in the hair follicle germ are not particularly limited as long as the epithelial cells have hair growth-related properties (e.g., the expression of a hair growth-related gene and/or the formation of a hair follicle germ through co-culture with mesenchymal cells). The epithelial cells may be cells derived from a hair follicle tissue (e.g., the outermost layer of the outer root sheath in the bulge region of a hair follicle tissue and/or the hair matrix portion), may be cells derived from skin tissue, or may be cells induced in vitro from stem cells (e.g., induced pluripotent stem (iPS) cells, embryonic stem (ES) cells, or embryonic germ (EG) cells). The epithelial cells may be primary cells collected from a living body, or may be cells cultured in advance (e.g., passaged cells and/or established cells).

The epithelial cells are identified as, for example, cells expressing a cytokeratin. The epithelial cells are preferably epithelial stem cells. The epithelial stem cells are identified as, for example, cells expressing cytokeratin 15 and/or CD34.

The epithelial cells are preferably derived from a human, but may be derived from a non-human animal (animal other than a human). The non-human animal is not particularly limited, but is preferably a non-human vertebrate (vertebrate other than a human). The non-human vertebrate is not particularly limited, but is preferably a non-human mammal. The non-human mammal is not particularly limited, but may be a primate (e.g., a monkey), a rodent (e.g., a mouse, a rat, a hamster, a guinea pig, or a rabbit), a carnivoran (e.g., a dog or a cat), or an ungulate (e.g., a pig, a bovine, a horse, a goat, or a sheep).

The mesenchymal cells contained in the hair follicle germ are not particularly limited as long as the mesenchymal cells have hair growth-related properties (e.g., the expression of a hair growth-related gene and/or the formation of a hair follicle germ through co-culture with epithelial cells). The mesenchymal cells may be cells derived from adult hair follicle tissue (e.g., dermal papilla and/or dermal sheath cup), may be cells derived from a skin tissue (which may be any of embryonic, juvenile, and adult skin tissues), or may be cells induced in vitro from stem cells (e.g., iPS cells, ES cells, or EG cells). The mesenchymal cells may be primary cells collected from a living body, or may be cells cultured in advance (e.g., passaged cells and/or established cells).

The mesenchymal cells are identified as, for example, cells expressing Versican and ALP. Specifically, the mesenchymal cells are preferably dermal papilla cells and/or dermal sheath cup cells. The dermal papilla cells and the dermal sheath cup cells express Versican and ALP.

The mesenchymal cells are preferably derived from a human, but may be derived from a non-human animal (animal other than a human). The non-human animal is not particularly limited, but is preferably a non-human vertebrate (vertebrate other than a human). The non-human vertebrate is not particularly limited, but is preferably a non-human mammal. The non-human mammal is not particularly limited, but may be a primate (e.g., a monkey), a rodent (e.g., a mouse, a rat, a hamster, a guinea pig, or a rabbit), a carnivoran (e.g., a dog or a cat), or an ungulate (e.g., a pig, a bovine, a horse, a goat, or a sheep).

The mesenchymal stem cells contained in the hair follicle germ are not particularly limited as long as the mesenchymal stem cells are somatic stem cells derived from a mesodermal tissue (mesenchyme). The mesenchymal stem cells may be, for example, bone marrow-derived mesenchymal stem cells, but are preferably adipose-derived mesenchymal stem cells (hereinafter referred to as "adipose-derived stem cells").

The adipose-derived stem cells may be mesenchymal stem cells derived from adipose tissue of a living body (which is not limited to subcutaneous fat, and may be any other adipose tissue), or may be cells induced in vitro from stem cells having an ability to differentiate into the adipose-derived stem cells (e.g., iPS cells, ES cells, or EG cells). The adipose-derived stem cells may be primary cells collected from a living body, or may be cells cultured in advance (e.g., passaged cells and/or established cells).

The adipose-derived stem cells are identified, in terms of adhesion property to a substrate in their culture system, as mesenchymal stem cells that adhere to the bottom surface (surface made of a resin, such as polystyrene) of a commercially available culture vessel. The adipose-derived stem cells are identified, in terms of their ability to differentiate, as, for example, mesenchymal stem cells having an ability to differentiate into bone cells, adipocytes, and chondrocytes (i.e., capable of differentiating into all of bone cells, adipocytes, and chondrocytes).

The adipose-derived stem cells are identified, in terms of their cell surface markers, as, for example, mesenchymal stem cells that are positive for CD73, CD90, and CD105. In this case, the adipose-derived stem cells may be identified as mesenchymal stem cells that are positive for CD29, CD44, CD73, CD90, CD105, and CD166. Further, in this case, the adipose-derived stem cells may be identified as mesenchymal stem cells that are positive for CD13, CD29, CD44, CD73, CD90, CD105, and CD166.

The adipose-derived stem cells may be identified as, in addition to being positive for the above-mentioned cell surface markers, mesenchymal stem cells that are negative for CD14, CD34, and CD45, or mesenchymal stem cells that are negative for CD14, CD31, and CD45. In this case, the adipose-derived stem cells may be identified as mesenchymal stem cells that are negative for CD14, CD19, CD34, CD45, and HLA-DR, or mesenchymal stem cells that are negative for CD14, CD31, CD34, and CD45. In addition, in the former case, the adipose-derived stem cells may be identified as mesenchymal stem cells that are negative for CD11b, CD14, CD19, CD34, CD45, CD79a, and HLA-DR.

The adipose-derived stem cells may be identified, in terms of their cell surface markers, on the basis of any combination of the above-mentioned positive markers and the above-mentioned negative markers. Specifically, the adipose-derived stem cells are identified as, for example, mesenchymal stem cells that are positive for CD73, CD90, and CD105, and negative for CD11b, CD14, CD19, CD34, CD45, CD79a, and HLA-DR. In addition, the adipose-derived stem cells are identified as, for example, mesenchymal stem cells that are positive for CD13, CD29, CD44, CD73, CD90, CD105, and CD166, and negative for CD14, CD31, and CD45. In addition, the adipose-derived stem cells are identified as, for example, mesenchymal stem cells that are positive for CD73, CD90, and CD105, and negative for CD14, CD19, CD34, CD45, and HLA-DR. In addition, the adipose-derived stem cells are identified as, for example, mesenchymal stem cells that are positive for CD29, CD44, CD73, CD90, CD105, and CD166, and negative for CD14, CD31, CD34, and CD45.

The adipose-derived stem cells are preferably derived from a human, but may be derived from a non-human animal (animal other than a human). The non-human animal is not particularly limited, but is preferably a non-human vertebrate (vertebrate other than a human). The non-human vertebrate is not particularly limited, but is preferably a non-human mammal. The non-human mammal is not particularly limited, but may be a primate (e.g., a monkey), a rodent (e.g., a mouse, a rat, a hamster, a guinea pig, or a rabbit), a carnivoran (e.g., a dog or a cat), or an ungulate (e.g., a pig, a bovine, a horse, a goat, or a sheep).

The respective numbers of the epithelial cells, the mesenchymal cells, and the mesenchymal stem cells contained in the hair follicle germ are not particularly limited as long as the numbers fall within a range in which the effect of the present invention is obtained, and are appropriately adjusted. That is, in the hair follicle germ, the ratio of the number of the mesenchymal cells to the number of the epithelial cells may be, for example, 0.01 or more, and is preferably 0.05 or more, more preferably 0.10 or more, still more preferably 0.15 or more, particularly preferably 0.20 or more.

The ratio of the number of the mesenchymal cells to the number of the epithelial cells may be, for example, 10.0 or less, 7.0 or less, or 5.0 or less. The ratio of the number of the mesenchymal cells to the number of the epithelial cells may be specified by arbitrarily combining any one of the above-mentioned lower limit values and any one of the above-mentioned upper limit values.

In the hair follicle germ, the ratio of the number of the mesenchymal stem cells to the total of the number of the epithelial cells and the number of the mesenchymal cells may be, for example, 0.01 or more, and is preferably 0.05 or more, more preferably 0.10 or more, still more preferably 0.15 or more, particularly preferably 0.20 or more.

The ratio of the number of the mesenchymal stem cells to the total of the number of the epithelial cells and the number of the mesenchymal cells may be, for example, 3.0 or less, or 1.5 or less. The ratio of the number of the mesenchymal stem cells to the total of the number of the epithelial cells and the number of the mesenchymal cells may be specified by arbitrarily combining any one of the above-mentioned lower limit values and any one of the above-mentioned upper limit values.

In the hair follicle germ, the ratio of the number of the mesenchymal stem cells to the number of the epithelial cells may be, for example, 0.02 or more, and is preferably 0.10 or more, more preferably 0.20 or more, still more preferably 0.30 or more, particularly preferably 0.40 or more.

The ratio of the number of the mesenchymal stem cells to the number of the epithelial cells may be, for example, 3.0 or less, or 1.5 or less. The ratio of the number of the mesenchymal stem cells to the number of the epithelial cells may be specified by arbitrarily combining any one of the above-mentioned lower limit values and any one of the above-mentioned upper limit values.

In the hair follicle germ, the ratio of the number of the mesenchymal stem cells to the number of the mesenchymal cells may be, for example, 0.02 or more, and is preferably 0.10 or more, more preferably 0.20 or more, still more preferably 0.30 or more, particularly preferably 0.40 or more.

The ratio of the number of the mesenchymal stem cells to the number of the mesenchymal cells may be, for example, 3.0 or less, or 1.5 or less. The ratio of the number of the mesenchymal stem cells to the number of the mesenchymal cells may be specified by arbitrarily combining any one of the above-mentioned lower limit values and any one of the above-mentioned upper limit values.

The hair follicle germ is formed by co-culturing epithelial cells, mesenchymal cells, and mesenchymal stem cells. That is, a method according to one embodiment of the present invention (hereinafter referred to as "method of the present invention") encompasses a method of producing a hair follicle germ, including co-culturing epithelial cells, mesenchymal cells, and mesenchymal stem cells to form a hair follicle germ. The co-culture is performed by mixing the epithelial cells, the mesenchymal cells, and the mesenchymal stem cells, and culturing the mixture.

A method for the co-culture is not particularly limited as long as the method allows the epithelial cells, the mesenchymal cells, and the mesenchymal stem cells to aggregate to form a hair follicle germ, but for example, it is preferred that the epithelial cells, the mesenchymal cells, and the mesenchymal stem cells be co-cultured on a non-cell-adhesive surface.

The non-cell-adhesive surface is not particularly limited as long as the epithelial cells, the mesenchymal cells, and the mesenchymal stem cells do not substantially adhere to the surface. That is, the non-cell-adhesive surface is, for example, such a surface that the epithelial cells, the mesenchymal cells, and the mesenchymal stem cells do not adhere thereto and are maintained in a suspended state, or such a surface that the epithelial cells, the mesenchymal cells, and the mesenchymal stem cells loosely adhere thereto, but the epithelial cells, the mesenchymal cells, and the mesenchymal stem cells are easily detached therefrom through an operation for fluidizing a culture solution, such as pipetting, without being subjected to enzymatic treatment, such as trypsin treatment. As a culture vessel having the non-cell-adhesive surface, there may be used, for example, a commercially available multiwell plate in which the bottom surface of each well is provided with a non-cell-adhesive coating. In addition, a microwell plate described in WO 2017/073625 A1 may also be preferably used.

When the epithelial cells, the mesenchymal cells, and the mesenchymal stem cells are co-cultured (e.g., the epithelial cells, the mesenchymal cells, and the mesenchymal stem cells are dispersed in a culture solution and cultured as a mixture), the epithelial cells, the mesenchymal cells, and the mesenchymal stem cells spontaneously aggregate to form a hair follicle germ. For example, the formed hair follicle germ includes an epithelial cell-aggregated portion formed through spontaneous aggregation of epithelial cells, and a mesenchymal cell-aggregated portion which is formed through spontaneous aggregation of mesenchymal cells, and a part of which is bound to a part of the epithelial cell-aggregated portion, and further includes mesenchymal stem cells present so as to be brought into contact with mesenchymal cells contained in the mesenchymal cell-aggregated portion.

In this case, mesenchymal stem cells numbering 50% or more, preferably 70% or more, of the total number of the mesenchymal stem cells contained in the hair follicle germ may be present so as to be brought into contact with the mesenchymal cell-aggregated portion (e.g., mesenchymal stem cells numbering 50% or more, preferably 70% or more, of the total number of the mesenchymal stem cells contained in the hair follicle germ are contained in the mesenchymal cell-aggregated portion). In addition, in each of those cases, mesenchymal stem cells numbering 50% or more, preferably 70% or more, of the total number of the mesenchymal stem cells contained in the hair follicle germ may be present without being brought into contact with the epithelial cell-aggregated portion (e.g., mesenchymal stem cells numbering 50% or more, preferably 70% or more, of the total number of the mesenchymal stem cells contained in the hair follicle germ are not contained in the epithelial cell-aggregated portion).

The hair follicle germ may be, for example, a so-called core-shell type hair follicle germ in which the mesenchymal cell-aggregated portion constitutes an inner core portion and the epithelial cell-aggregated portion constitutes an outer layer covering the outer periphery of the mesenchymal cell-aggregated portion. The hair follicle germ, for example, may include the mesenchymal cell-aggregated portion and the epithelial cell-aggregated portion that are adjacent to each other, where a part of the outer surface of the mesenchymal cell-aggregated portion and a part of the outer surface of the epithelial cell-aggregated portion are in contact with each other. While the hair follicle germ preferably includes the mesenchymal cell-aggregated portion and the epithelial cell-aggregated portion as described above, the hair follicle germ may be, for example, a so-called random type hair follicle germ in which the mesenchymal cells and the epithelial cells are arranged in a dispersed manner. In each of those hair follicle germs, at least some of the mesenchymal stem cells are in contact with the mesenchymal cells as described above.

On the non-cell-adhesive surface, a hair follicle germ in a non-adherent state is produced. Herein, the hair follicle germ in a non-adherent state is a hair follicle germ in a suspended state without adhering to the non-cell-adhesive surface, or a hair follicle germ loosely adhering to the non-cell-adhesive surface but easily detachable from the non-cell-adhesive surface through an operation for fluidizing a culture solution, such as pipetting, without being subjected to enzymatic treatment, such as trypsin treatment.

The hair follicle germ may be a hair follicle germ spheroid. The hair follicle germ spheroid is an approximately spherical cell aggregate. The hair follicle germ spheroid is formed in a non-adherent state, and hence is easily collected. For this reason, the hair follicle germ spheroid is preferably used for transplantation to a living body.

The hair follicle germ formed through the co-culture including the mesenchymal stem cells contains, for example, mesenchymal cells that have been activated compared to mesenchymal cells contained in a hair follicle germ formed by the same method except for omitting use of the mesenchymal stem cells.

The activation of the mesenchymal cells means the improvement of the hair growth-related properties of the mesenchymal cells. Specifically, the improvement of the hair growth-related properties of the mesenchymal cells includes, for example, an increase in expression amount of a hair growth-related gene in the mesenchymal cells and/or the improvement of the hair growth properties of the hair follicle germ formed using the mesenchymal cells (e.g., increase in the number and/or lengths of hairs grown from the hair follicle germ transplanted to a living body).

That is, for example, the mesenchymal cells activated through co-culture with the mesenchymal stem cells may have an increased expression amount of at least one hair growth-related gene compared to that of mesenchymal cells contained in a hair follicle germ formed by the same method except for omitting use of the mesenchymal stem cells.

The hair growth-related gene to be expressed in the mesenchymal cells is not particularly limited as long as the gene is related to the contribution of the mesenchymal cells to hair growth, but may be, for example, one or more selected from the group consisting of Versican, ALP, BMP4, Nexin, and Notch1.

Specifically, in the activated mesenchymal cells contained in the hair follicle germ containing the mesenchymal stem cells, for example, the expression amount of a Versican gene measured by RT-PCR may be 1.2 times or more, preferably 1.5 times or more, more preferably 1.8 times or more, particularly preferably 2.0 times or more, larger than that in mesenchymal cells contained in a hair follicle germ formed by the same method except for omitting use of the mesenchymal stem cells.

In the activated mesenchymal cells contained in the hair follicle germ containing the mesenchymal stem cells, for example, the expression amount of an ALP gene measured by RT-PCR may be 1.2 times or more, preferably 1.5 times or more, particularly preferably 1.7 times or more, larger than that in mesenchymal cells contained in a hair follicle germ formed by the same method except for omitting use of the mesenchymal stem cells.

In the activated mesenchymal cells contained in the hair follicle germ containing the mesenchymal stem cells, for example, the expression amount of a BMP4 gene measured by RT-PCR may be 1.1 times or more, preferably 1.2 times or more, particularly preferably 1.3 times or more, larger than that in mesenchymal cells contained in a hair follicle germ formed by the same method except for omitting use of the mesenchymal stem cells.

The hair follicle germ formed through the co-culture including the mesenchymal stem cells contains, for example, epithelial cells that have been activated compared to epithelial cells contained in a hair follicle germ formed by the same method except for omitting use of the mesenchymal stem cells.

The activation of the epithelial cells means the improvement of the hair growth-related properties of the epithelial cells. Specifically, the improvement of the hair growth-related properties of the epithelial cells includes, for example, an increase in expression amount of a hair growth-related gene in the epithelial cells and/or the improvement of the hair growth properties of the hair follicle germ formed using the epithelial cells (e.g., increase in the number and/or lengths of hairs grown from the hair follicle germ transplanted to a living body).

That is, for example, the epithelial cells activated through co-culture with the mesenchymal stem cells may have an increased expression amount of at least one hair growth-related gene compared to that of epithelial cells contained in a hair follicle germ formed by the same method except for omitting use of the mesenchymal stem cells.

The hair growth-related gene to be expressed in the epithelial cells is not particularly limited as long as the gene is related to the contribution of the epithelial cells to hair growth, but may be, for example, one or more selected from the group consisting of Wnt10b, LEF1, Shh, MSX2, and β-Catenin.

Specifically, in the activated epithelial cells contained in the hair follicle germ containing the mesenchymal stem cells, for example, the expression amount of a hair growth-related gene (e.g., one or more selected from the group consisting of Wnt10b, LEF1, Shh, MSX2, and β-Catenin) measured by RT-PCR is larger than that of mesenchymal cells contained in a hair follicle germ formed by the same method except for omitting use of the mesenchymal stem cells.

Consequently, the production of the hair follicle germ by the method of the present invention may be the production of a hair follicle germ containing activated mesenchymal cells, that is, the production of an activated hair follicle germ. Accordingly, the method of the present invention encompasses a method of activating mesenchymal cells, including, in a hair follicle germ formed by co-culturing epithelial cells and mesenchymal cells, performing the co-culture with further addition of mesenchymal stem cells, to thereby activate the mesenchymal cells contained in the hair follicle germ.

The hair follicle germ according to this embodiment is preferably used for transplantation to a living body. That is, according to the method of the present invention, a hair follicle germ that causes hair growth when transplanted to a living body is produced.

When the hair follicle germ is transplanted to a living body to cause hair growth from the hair follicle germ, the living body is not particularly limited, and may be a human or a non-human animal. The non-human animal is not particularly limited, but is preferably a non-human mammal. The non-human mammal is not particularly limited, but may be, for example, a primate (e.g., a monkey), a rodent (e.g., a mouse, a rat, a hamster, a guinea pig, or a rabbit), a carnivoran (e.g., a dog or a cat), or an ungulate (e.g., a pig, a bovine, a horse, a goat, or a sheep). The transplantation of the hair follicle germ to a living body is preferably transplantation to the skin of the living body. The transplantation to the skin may be, for example, subcutaneous transplantation or intradermal transplantation.

The production of the hair follicle germ and the transplantation thereof to an animal may be applied to medicine or research. That is, for example, for the purpose of treating or preventing a disease associated with hair loss, the hair follicle germ may be produced for being transplanted to a human patient suffering from or at a risk of suffering from the disease, or the hair follicle germ may be transplanted to the human patient.

The disease associated with hair loss is not particularly limited, but may be, for example, one or more selected from the group consisting of androgenetic alopecia (AGA), female androgenetic alopecia (FAGA), postpartum alopecia, diffuse alopecia, seborrheic alopecia, alopecia pityrodes, traction alopecia, metabolic alopecia, pressure alopecia, alopecia areata, alopecia neurotica, hair-pulling disorder, universalis alopecia, and symptomatic alopecia.

In addition, for example, for the purpose of searching for a substance that may be used for the treatment or prevention of a disease associated with hair loss and/or searching for a substance involved in the mechanism of the disease, the hair follicle germ may be produced, or the hair follicle germ may be transplanted to a non-human animal.

Next, specific Examples according to the embodiments of the present invention will be described.

### Example 1

### [Collection of Epithelial Cells]

A dorsal skin tissue was collected from a C57BL/6 mouse embryo at embryonic day 18, and was subjected to dispase treatment by a partially modified version of a method reported by Nakao et al. (Koh-ei Toyoshima et al. Nature Communications, 3, 784, 2012) at 4°C under a shaking condition of 30 rpm for 1 hour to separate the epithelial layer and mesenchymal layer of the skin tissue. After that, the epithelial layer was treated with 100 U/mL collagenase for 1 hour and 20 minutes and further treated with trypsin for 10 minutes to isolate epithelial cells.

### [Preparation of Mesenchymal Cells]

Dermal papilla cells were used as mesenchymal cells. That is, commercially available human dermal papilla cells (PromoCell GmbH) were passaged, and the human dermal papilla cells on passages 4 to 6 (P4 to P6) were used. A commercially available culture medium for dermal papilla cells (PromoCell GmbH) was used as a culture solution.

### [Preparation of Mesenchymal Stem Cells]

Adipose-derived stem cells were used as mesenchymal stem cells. That is, commercially available human adipose-derived stem cells (Lonza) were passaged, and the human adipose-derived stem cells on passages 4 to 6 (P4 to P6) were used. A commercially available DMEM culture medium (Sigma) was used as a culture solution.

### [Production of Hair Follicle Germs]

A commercially available 96 multiwell plate in which each well had a round bottom provided with a non-cell-adhesive coating (non-cell-adhesive bottom surface curved in a concave shape) was used as a culture vessel for forming hair follicle germs.

In the production, each well was inoculated with a mixture of the epithelial cells and the dermal papilla cells each at 4×10³ cells/well, and the adipose-derived stem cells at 2×10³ cells/well (total number of cells: 1×10⁴ cells/well), and the cells were co-cultured for 3 days. A mixed medium prepared by mixing a dermal papilla cell culture medium (Follicle Dermal Papilla Cell Growth Medium kit, PromoCell GmbH) and HuMedia-KG2 at a volume ratio of 1:1 was used as a culture solution.

### [Results]

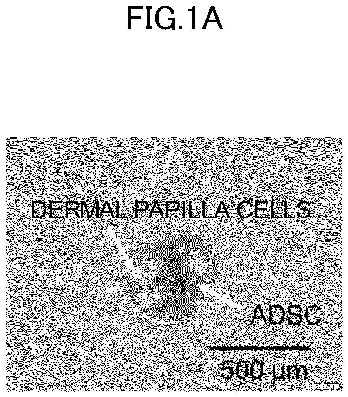

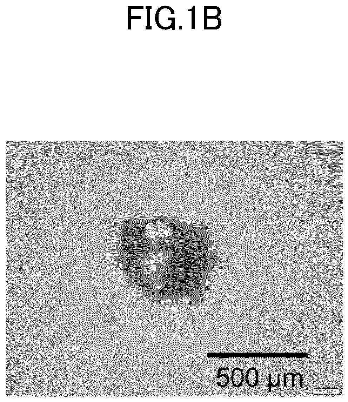

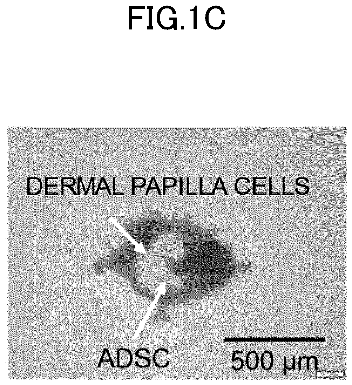

FIG. 1A, FIG. 1B, and FIG. 1C show fluorescence micrographs of a hair follicle germ formed in one well on day 1 of culture, day 2 of culture, and day 3 of culture, respectively. In the hair follicle germ shown in FIG. 1A to FIG. 1C, the dermal papilla cells were fluorescently stained green with a fluorescent reagent Vybrant DiO, and the adipose-derived stem cells ("ADSC" in the figures) were fluorescently stained red with a fluorescent reagent Vybrant Dil.

As shown in FIG. 1A, FIG. 1B, and FIG. 1C, as the culture time progressed, the epithelial cells spontaneously aggregated with each other to form an epithelial cell-aggregated portion, and the dermal papilla cells also spontaneously aggregated with each other to form a dermal papilla cell-aggregated portion, and consequently a hair follicle germ spheroid was formed. In the hair follicle germ spheroid, many of the adipose-derived stem cells were distributed in the dermal papilla cell-aggregated portion.

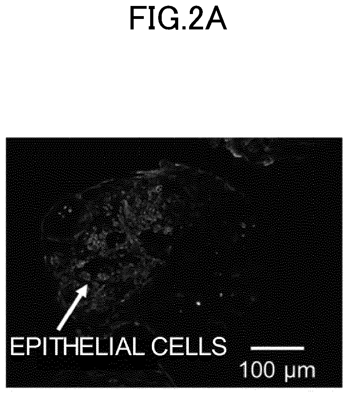

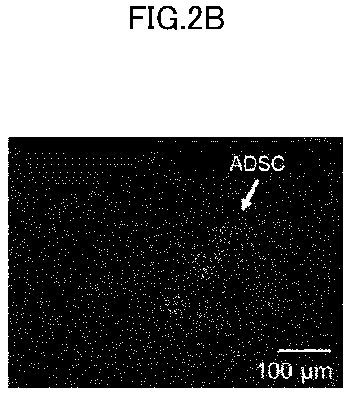

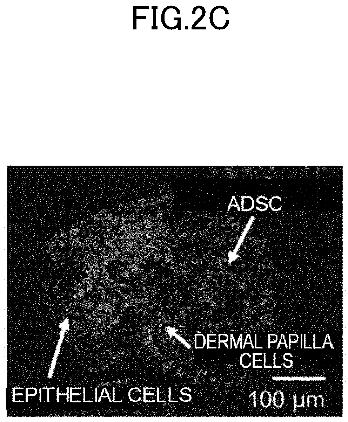

FIG. 2A, FIG. 2B, and FIG. 2C show fluorescence micrographs of the hair follicle germ formed in one well on day 3 of culture. In the hair follicle germ, the epithelial cells were fluorescently stained green via an anti-cytokeratin 15 antibody, the cell nuclei of all the cells were fluorescently stained blue with a fluorescent reagent DAPI, and the adipose-derived stem cells were fluorescently stained red with a fluorescent reagent Vybrant Dil. FIG. 2A shows a green fluorescence image corresponding to the epithelial cells, FIG. 2B shows a red fluorescence image corresponding to the adipose-derived stem cells, and FIG. 2C shows a fluorescence image of three colors corresponding to the epithelial cells, the dermal papilla cells, and the adipose-derived stem cells.

As shown in FIG. 2A, the epithelial cells formed an epithelial cell-aggregated portion as a part of the hair follicle germ. On the other hand, as shown in FIG. 2C, the dermal papilla cells formed a dermal papilla cell-aggregated portion as another part of the hair follicle germ. The epithelial cell-aggregated portion and the dermal papilla cell-aggregated portion were adjacently formed so that a part of the epithelial cell-aggregated portion and a part of the dermal papilla cell-aggregated portion were in contact with each other. In addition, as shown in FIG. 2B and FIG. 2C, many of the adipose-derived stem cells were present in the dermal papilla cell-aggregated portion.

### Example 2

### [Collection of Epithelial Cells]

Epithelial cells were isolated from skin tissue of a C57BL/6 mouse embryo at embryonic day 18 in the same manner as in Example 1 described above.

### [Preparation of Mesenchymal Cells]

Dermal papilla cells were prepared as mesenchymal cells in the same manner as in Example 1 described above.

### [Preparation of Mesenchymal Stem Cells]

Adipose-derived stem cells were prepared as mesenchymal stem cells in the same manner as in Example 1 described above.

### [Production of Hair Follicle Germs]

A 96 multiwell plate was used as a culture vessel, as with Example 1 described above. Then, the epithelial cells, the dermal papilla cells, and the adipose-derived stem cells were co-cultured in each well using the mixed medium in the same manner as in Example 1 described above, with the ratio among the inoculated numbers of the epithelial cells, the dermal papilla cells, and the adipose-derived stem cells being varied.

Specifically, in Example 2-1, co-culture was performed at a number ratio of epithelial cells:dermal papilla cells:adipose-derived stem cells=2:2:1. That is, each well was inoculated with a mixture of the epithelial cells and the dermal papilla cells each at 4×10³ cells/well, and the adipose-derived stem cells at 2×10³ cells/well (total number of cells: 1×10⁴ cells/well), and the cells were co-cultured for 3 days.

Similarly, in Example 2-2, co-culture was performed at a number ratio of epithelial cells:dermal papilla cells:adipose-derived stem cells=4:4:1. That is, each well was inoculated with a mixture of the epithelial cells and the dermal papilla cells each at 4×10³ cells/well, and the adipose-derived stem cells at 1×10³ cells/well (total number of cells: 9×10³ cells/well), and the cells were co-cultured for 3 days.

In addition, in Example 2-3, co-culture was performed at a number ratio of epithelial cells:dermal papilla cells:adipose-derived stem cells=8:8:1. That is, each well was inoculated with a mixture of the epithelial cells and the dermal papilla cells each at 4×10³ cells/well, and the adipose-derived stem cells at 0.5×10³ cells/well (total number of cells: 8.5×10³ cells/well), and the cells were co-cultured for 3 days.

On the other hand, in Comparative Example 2-1, co-culture was performed at a number ratio of epithelial cells:dermal papilla cells:adipose-derived stem cells=1:1:0. That is, without the use of the adipose-derived stem cells, each well was inoculated with a mixture of the epithelial cells and the dermal papilla cells each at 4×10³ cells/well (total number of cells: 8×10³ cells/well), and the cells were co-cultured for 3 days.

### [PCR Analysis of Hair Growth-related Genes]

Gene expression amounts in hair follicle germs formed through the 3 days of culture were analyzed by RT-PCR. The base sequences of primers used in RT-PCR were as follows: Forward Primer for Versican: "5'-GCTGCAAAAGAGTGTGAAAA-3"' and Reverse Primer therefor: "5'-AGTGGTAACGAGATGCTTCC-3'" ; Forward Primer for BMP4: "5'-GCCCGCAGCCTAGCAA-3"' and Reverse Primer therefor: "5'-CGGTAAAGATCCCGCATGTAG-3'"; Forward Primer for ALP: "5'-ATTGACCACGGGCACCAT-3'" and Reverse Primer therefor: "5'-CTCCACCGCCTCAGCA-3'"; and Forward Primer for GAPDH used as a control: "5'-ACCACAGTCCATGCCATCAC-3'" and Reverse Primer therefor: "5'-TCCACCACCCTGTTGCTGTA-3'".

### [Results]

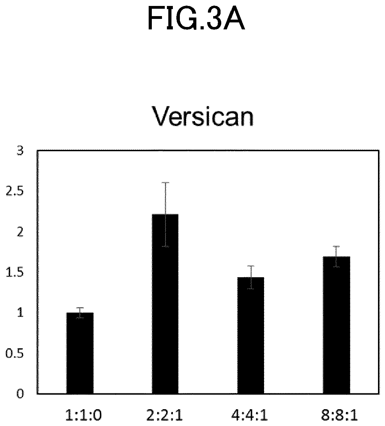

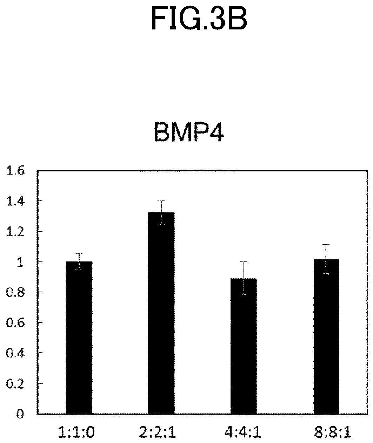

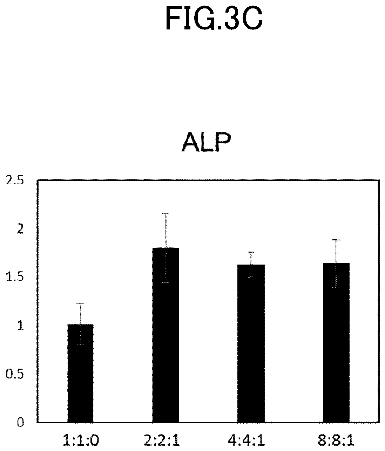

FIG. 3A, FIG. 3B, and FIG. 3C show the results of the analysis of the expression amounts of the Versican gene, the BMP4 gene, and the ALP gene, respectively, in the hair follicle germs formed through co-culture in which the ratio among the inoculated numbers of the epithelial cells, the dermal papilla cells, and the adipose-derived stem cells was varied. Specifically, FIG. 3A, FIG. 3B, and FIG. 3C each show the relative gene expression amount (vertical axis of the figure) of each of the hair follicle germ of Comparative Example 2-1 formed without using the adipose-derived stem cells ("1:1:0" in the figures), the hair follicle germ of Example 2-1 formed at a number ratio of epithelial cells:dermal papilla cells:adipose-derived stem cells=2:2:1 ("2:2:1" in the figures), the hair follicle germ of Example 2-2 formed at a number ratio of epithelial cells:dermal papilla cells:adipose-derived stem cells=4:4:1 ("4:4:1" in the figures), and the hair follicle germ of Example 2-3 formed at a number ratio of epithelial cells:dermal papilla cells:adipose-derived stem cells=8:8:1 ("8:8:1" in the figures), in the case where the gene expression amount in the Comparative Example 2-1 is defined as "1".

As shown in FIG. 3A and FIG. 3C, in all the hair follicle germs formed using the adipose-derived stem cells, the expression amounts of the Versican gene and the ALP gene were increased compared to those in the hair follicle germ formed without using the adipose-derived stem cells ("1:1:0" in the figures). In particular, the expression amounts of the Versican gene and the ALP gene in the hair follicle germ formed at a number ratio of epithelial cells:dermal papilla cells:adipose-derived stem cells=2:2:1 ("2:2:1" in the figures) were remarkably large.

In addition, as shown in FIG. 3B, with regard to the BMP4 gene, increases in expression amount were not found in Example 2-2 ("4:4:1" in the figure) and Example 2-3 ("8:8:1" in the figure), but in Example 2-1 ("2:2:1" in the figure) having a relatively large ratio of the adipose-derived stem cells, the expression amount of the BMP4 gene was remarkably increased compared to that in the hair follicle germ formed without using the adipose-derived stem cells ("1:1:0" in the figure).

That is, it was recognized that the expression amounts of a hair growth-related genes in the hair follicle germ (specifically, the amounts of the Versican gene, the BMP4 gene, and the ALP gene expressed by the dermal papilla cells contained in the hair follicle germ) can be effectively increased by performing the co-culture using the adipose-derived stem cells in addition to the epithelial cells and the dermal papilla cells to form the hair follicle germ.

### Example 3

### [Production of Multiwell Culture Vessel]

A multiwell culture vessel to be used for co-culture for forming hair follicle germ spheroids was produced in the same manner as in Patent Literature 1 described above. That is, first, through use of CAD software (V Carve Pro 6.5), the pattern of multiple wells to be produced was designed on a computer. Subsequently, through use of a cutting machine, an olefin-based resin substrate was cut according to the designed pattern to produce a concave mold having the multiple wells pattern that was designed. An epoxy resin (CRYSTAL RESIN, manufactured by Nissin Resin Co., Ltd.) was poured into the mold, and cured for 1 day. After that, the mold was released to form a convex mold having the above-mentioned designed pattern. The formed convex mold was fixed to the bottom surface of a 24-well plate, and polydimethylsiloxane (PDMS) was poured into the plate and solidified. After that, the mold was released. Thus, a multiwell culture vessel (hereinafter referred to as "PDMS spheroid chip") having multiple wells (each well having a diameter of 1 mm and a depth of 1 mm) formed in a regular pattern on a PDMS substrate was produced.

In the PDMS spheroid chip, the wells are formed in the substrate made of PDMS having excellent oxygen permeability, and hence cells and cell aggregates cultured in the wells are supplied with an appropriate amount of oxygen throughout a culture period.

### [Collection of Epithelial Cells]

Epithelial cells were isolated from skin tissue of a C57BL/6 mouse embryo at embryonic day 18 in the same manner as in Example 1 described above.

### [Preparation of Mesenchymal Cells]

Dermal papilla cells were prepared as mesenchymal cells in the same manner as in Example 1 described above.

### [Preparation of Mesenchymal Stem Cells]

Adipose-derived stem cells were prepared as mesenchymal stem cells in the same manner as in Example 1 described above.

### [Production of Large Quantity of Hair Follicle Germs]

The PDMS spheroid chip produced as described above was used as a culture vessel. Each well of the PDMS spheroid chip was inoculated with a mixture of the epithelial cells and the dermal papilla cells each at 4×10³ cells/well, and the adipose-derived stem cells at 2×10³ cells/well (total number of cells: 1×10⁴ cells/well), and the cells were co-cultured for 3 days. A mixed medium prepared by mixing a dermal papilla cell culture medium (Follicle Dermal Papilla Cell Growth Medium kit, PromoCell GmbH) and HuMedia-KG2 at a volume ratio of 1:1 was used as a culture solution.

### [Results]

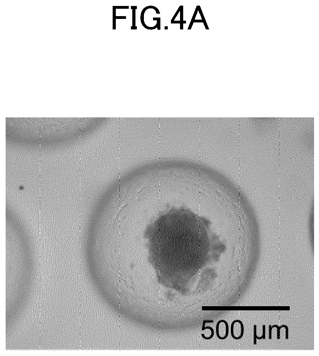

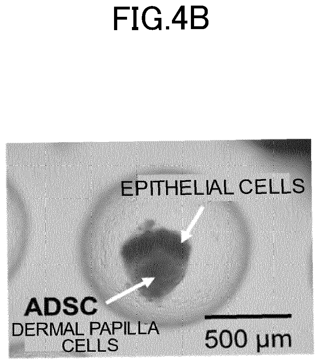

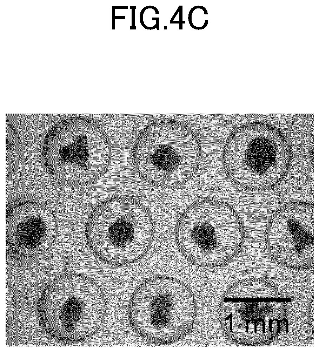

FIG. 4A and FIG. 4B show high-magnification phase-contrast micrographs of a hair follicle germ formed in a well on day 1 of culture and day 3 of culture, respectively, and FIG. 4C shows a low-magnification phase-contrast micrograph of hair follicle germs formed in wells on day 3 of culture.

As shown in FIG. 4A to FIG. 4C, a hair follicle germ was formed by co-culturing the epithelial cells, the dermal papilla cells, and the adipose-derived stem cells in each well of the PDMS spheroid chip. As shown in FIG. 4B, the hair follicle germ included an epithelial cell-aggregated portion formed through aggregation of the epithelial cells and a dermal papilla cell-aggregated portion formed through aggregation of the dermal papilla cells, and many of the adipose-derived stem cells ("ADSC" in the figure) were present in the dermal papilla cell-aggregated portion. In addition, as shown in FIG. 4C, it was recognized that the use of the PDMS spheroid chip can achieve simultaneous production of a large quantity of hair follicle germs having a uniform size.

### Example 4

### [Collection of Epithelial Cells]

Epithelial cells were isolated from a skin tissue of a C57BL/6 mouse embryo at embryonic day 18 in the same manner as in Example 1 described above.

### [Preparation of Mesenchymal Cells]

Hermal papilla cells were prepared as mesenchymal cells in the same manner as in Example 1 described above.

### [Preparation of Mesenchymal Stem Cells]

Adipose-derived stem cells were prepared as mesenchymal stem cells in the same manner as in Example 1 described above.

### [Production of Hair Follicle Germs]

A 96 multiwell plate was used as a culture vessel as with Example 1 described above. Then, the epithelial cells, the dermal papilla cells, and the adipose-derived stem cells were co-cultured in each well with the ratio among the inoculated numbers of the epithelial cells, the dermal papilla cells, and the adipose-derived stem cells being varied. A mixed medium prepared by mixing a dermal papilla cell culture medium (Follicle Dermal Papilla Cell Growth Medium kit, PromoCell GmbH) and HuMedia-KG2 at a volume ratio of 1:1 was used as a culture solution.

Specifically, in Example 4-1, co-culture was performed at a number ratio of epithelial cells:dermal papilla cells:adipose-derived stem cells=2:2:1. That is, each well was inoculated with a mixture of the epithelial cells and the dermal papilla cells each at 4×10³ cells/well, and the adipose-derived stem cells at 2×10³ cells/well (total number of cells: 1×10⁴ cells/well), and the cells were co-cultured for 3 days.

Similarly, in Example 4-2, co-culture was performed at a number ratio of epithelial cells:dermal papilla cells:adipose-derived stem cells=4:4:1. That is, each well was inoculated with a mixture of the epithelial cells and the dermal papilla cells each at 4×10³ cells/well, and the adipose-derived stem cells at 1×10³ cells/well (total number of cells: 9×10³ cells/well), and the cells were co-cultured for 3 days.

On the other hand, in Comparative Example 4-1, co-culture was performed at a number ratio of epithelial cells:dermal papilla cells:adipose-derived stem cells=1:1:0. That is, without the use of the adipose-derived stem cells, each well was inoculated with a mixture of the epithelial cells and the dermal papilla cells each at 4×10³ cells/well (total number of cells: 8×10³ cells/well), and the cells were co-cultured for 3 days.

### [Transplantation to Mice]

Hair follicle germs formed as described above were collected on day 3 of culture and intradermally transplanted to nude mice. That is, the nude mice were anesthetized through inhalation of isoflurane, and the dorsal part thereof was disinfected with Isodine. Subsequently, a V-lance micro-scalpel (Alcon Japan Ltd.) was used to form incisions for transplantation ranging from the epidermal layer of the skin to a lower part of the dermal layer. Then, the incisions for transplantation were each injected with 20 of the hair follicle germs. The care of the nude mice and the transplantation experiment were performed in conformity with the guidelines of the expert committee on animal experiments at Yokohama National University.

### [Results]

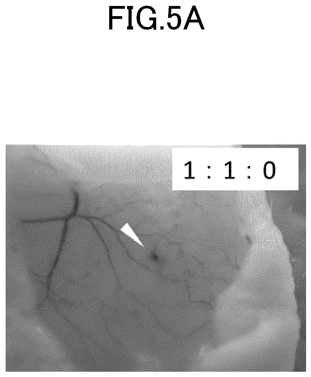

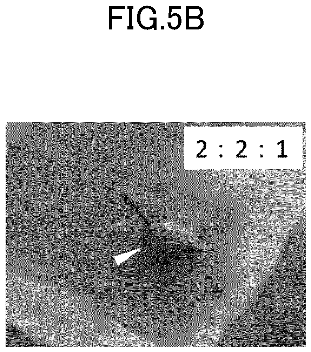

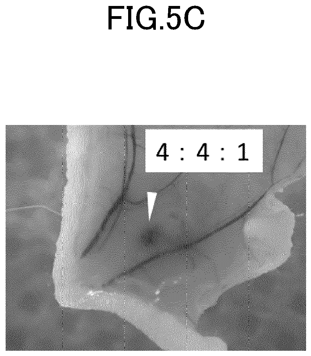

FIG. 5A, FIG. 5B, and FIG. 5C respectively show photographs of hair regenerated from the hair follicle germs of Comparative Example 2-1 formed without using the adipose-derived stem cells ("1:1:0" in FIG. 5A), the hair follicle germs of Example 4-1 formed at a number ratio of epithelial cells:dermal papilla cells:adipose-derived stem cells=2:2:1 ("2:2:1" in FIG. 5B), and the hair follicle germs of Example 4-2 formed at a number ratio of epithelial cells:dermal papilla cells:adipose-derived stem cells=4:4:1 ("4:4:1" in FIG. 5C). The photographs were taken at 30 days after the transplantation of the hair follicle germs to the mice.

As shown in FIG. 5A, hair growth from the hair follicle germs containing no adipose-derived stem cells was very slight. On the other hand, as shown in FIG. 5C, the quantity of hair growth from the hair follicle germs formed at a number ratio of epithelial cells:dermal papilla cells:adipose-derived stem cells=4:4:1 was large compared to that of the hair follicle germs containing no adipose-derived stem cells. Further, as shown in FIG. 5B, hair growth from the hair follicle germs formed at a number ratio of epithelial cells:dermal papilla cells:adipose-derived stem cells=2:2:1 was particularly remarkable, and clear hair growth was found.

That is, it was recognized that the hair growth ability of a hair follicle germ can be improved when the adipose-derived stem cells are added to the epithelial cells and the dermal papilla cells in co-culture for forming the hair follicle germ.

## Claims

1. A hair follicle germ, comprising:
epithelial cells;
mesenchymal cells; and
mesenchymal stem cells.

2. The hair follicle germ according to claim 1, wherein the mesenchymal stem cells are adipose-derived mesenchymal stem cells.

3. The hair follicle germ according to claim 1 or 2, wherein the mesenchymal cells are dermal papilla cells and/or dermal sheath cup cells.

4. The hair follicle germ according to any one of claims 1 to 3, wherein the hair follicle germ is a hair follicle germ spheroid.

5. The hair follicle germ according to any one of claims 1 to 4, wherein a ratio of the number of the mesenchymal stem cells to a total of the number of the epithelial cells and the number of the mesenchymal cells is 0.01 or more.

6. The hair follicle germ according to any one of claims 1 to 5, wherein a ratio of the number of the mesenchymal stem cells to the number of the epithelial cells is 0.02 or more.

7. The hair follicle germ according to any one of claims 1 to 6, wherein a ratio of the number of the mesenchymal stem cells to the number of the mesenchymal cells is 0.02 or more.

8. A method of producing a hair follicle germ, comprising co-culturing epithelial cells, mesenchymal cells, and mesenchymal stem cells to form a hair follicle germ.

9. The method of producing a hair follicle germ according to claim 8, wherein the co-culturing comprises co-culturing the epithelial cells, the mesenchymal cells, and the mesenchymal stem cells on a non-cell-adhesive surface.

10. The method of producing a hair follicle germ according to claim 8 or 9, wherein the hair follicle germ formed by the co-culture contains the mesenchymal cells that have been activated compared to mesenchymal cells contained in a hair follicle germ formed by the same method except for omitting use of the mesenchymal stem cells.

11. The method of producing a hair follicle germ according to claim 10, wherein, in the mesenchymal cells activated, an expression amount of at least one hair growth-related gene is increased compared to that in mesenchymal cells contained in a hair follicle germ formed by the same method except for omitting use of the mesenchymal stem cells.

12. A method of activating mesenchymal cells, comprising, in a hair follicle germ formed by co-culturing epithelial cells and mesenchymal cells, performing the co-culture with further addition of mesenchymal stem cells, to thereby activate the mesenchymal cells contained in the hair follicle germ.


SEQUENCE LISTING

<110>  National University Corporation YOKOHAMA National University
       KANAGAWA INSTITUTE OF INDUSTRIAL SCIENCE AND TECHNOLOGY

<120>  Hair Follicle Primordium

<130>  F1903PCT

<160>  4

<170>  PatentIn version 3.5

<210>  1
<211>  20
<212>  DNA
<213>  Artificial Sequence

<220>
<223>  Versican F

<400>  1
gctgcaaaag agtgtgaaaa                                                  20


<210>  2
<211>  20
<212>  DNA
<213>  Artificial Sequence

<220>
<223>  Versican R

<400>  2
agtggtaacg agatgcttcc                                                  20


<210>  3
<211>  20
<212>  DNA
<213>  Artificial Sequence

<220>
<223>  GAPDH F

<400>  3
agaacatcat ccctgcatcc                                                  20


<210>  4
<211>  20
<212>  DNA
<213>  Artificial Sequence

<220>
<223>  GAPDH R

<400>  4
tccaccaccc tgttgctgta                                                  20


<210>  5
<211>  16

<212>  DNA
<213>  Artificial Sequence

<220>
<223>  BMP4 F

<400>  5
gcccgcagcc tagcaa                                                        16


<210>  6
<211>  21
<212>  DNA
<213>  Artificial Sequence

<220>
<223>  BMP4 R

<400>  6
cggtaaagat cccgcatgtag                                                   21


<210>  7
<211>  18
<212>  DNA
<213>  Artificial Sequence

<220>
<223>  ALP F

<400>  7
attgaccacg ggcaccat                                                      18


<210>  8
<211>  16
<212>  DNA
<213>  Artificial Sequence

<220>
<223>  ALP R

<400>  8
ctccaccgcc tcagca                                                        16